# EUROPÄISCHE PATENTANMELDUNG

(11) **EP 1 837 632 A2**
(43) Veröffentlichungstag der Anmeldung: **26.09.2007**
(21) Anmeldenummer: 07004399.7
(22) Anmeldetag: 03.03.2007
(51) Int. Cl.: G01F 1/075

(54) **Einrichtung zur Erfassung des Vorhandenseins eines oder mehrerer Stoffe in einem in einer Rohrleitung strömenden Medium**

(30) Priorität: 22.03.2006 DE 102006013613
(71) Anmelder: Hydrometer GmbH, 91522 Ansbach (DE)
(72) Erfinder: Schulze, Manfred, 91522 Ansbach (DE)
(74) Vertreter: Blaumeier, Jörg

(57) **Zusammenfassung**

Einrichtung zur Erfassung des Vorhandenseins eines oder mehrerer Stoffe oder Verbindungen in einem in einer Rohrleitung strömenden Medium, insbesondere Wasser, wobei durch eine der Erfassung der Stoffe oder Verbindungen dienende Sensoreinrichtung (13), die in einem in die Rohrleitung zu setzenden Gehäuse (3) angeordnet ist, und von einem das Medium führenden Strömungskanal (5) über einen eine Gasdiffusion aus dem Medium erlaubenden Diffusionsabschnitt (16) getrennt ist.

## Beschreibung

Die Erfindung betrifft eine Einrichtung zur Erfassung des Vorhandenseins eines oder mehrerer Stoffe oder Verbindungen in einem in einer Rohrleitung strömenden Medium, insbesondere Wasser.

Trinkwasser ist das wichtigste Nahrungsmittel für den Menschen, weshalb die Einhaltung der in Gesetzen und Verordnungen festgelegten Grenzwerte sowie die frühzeitige Erkennung von Schadstoffen und Gefahrstoffen im Wasser von besonders hoher Bedeutung ist. Bisher erfolgt die Reinheitsprüfung von Wasser der Gestalt, dass an bestimmten Messstellen manuell Wasserproben entnommen werden, die anschließend in einem Labor auf ihren Inhalt hin analysiert werden. Dies ist nicht nur umständlich, sondern auch sehr zeitaufwendig. Vom Entnehmen der Probe bis hin zum endgültigen Analyseergebnis vergeht oft eine geraume Zeit, so dass sich die ggf. erfasste bedenkliche Situation bereits wieder deutlich geändert haben kann.

Der Erfindung liegt damit das Problem zugrunde, eine Einrichtung anzugeben, die auf einfache Weise die Erfassung etwaiger bedenklicher Stoffe oder Verbindungen in einem zu überwachenden Medium, vornehmlich Wasser, zulässt.

Zur Lösung dieses Problems zeichnet sich eine Einrichtung der eingangs genannten Art durch eine der Erfassung der Stoffe oder Verbindungen dienendes Sensoreinrichtung aus, die in einem in die Rohrleitung zu setzenden Gehäuse angeordnet ist, und von einem das Medium führenden Strömungskanal über einen eine Gasdiffusion aus dem Medium erlaubenden Diffusionsabschnitt getrennt ist.

Die erfindungsgemäße Einrichtung wird über das entsprechend ausgeführte Gehäuse unmittelbar in eine Rohrleitung, in der das zu überwachende Medium strömt, gesetzt. Dies bietet folglich die Möglichkeit, vor Ort, kontinuierlich und in situ das Medium, vornehmlich Wasser, auf seine Inhaltsstoffe zumindest grob analysieren zu können. Die Sensoreinrichtung, die beispielsweise in Form eines Sensors oder Arrays auf Basis von Metalloxiden oder leitenden Polymeren ausgeführt ist und für eine beliebige Vielzahl verschiedener Stoffe oder Verbindungen sensitiv sein kann, stellt das Erfassungsergebnis unmittelbar zur Verfügung, wobei das Erfassungsergebnis bevorzugt über eine ihr zugeordnete Datenübertragungseinrichtung an eine Datenempfangseinrichtung übertragen werden kann. Diese Datenübertragungseinrichtung kann als Funkeinrichtung ausgeführt sein oder eine drahtgebundene Kommunikation zu einer externen Datenempfangseinrichtung zulassen. In jedem Fall besteht die Möglichkeit, lokal vor Ort unmittelbar das Wasser auf etwaige gefährdende Inhaltsstoffe hin zu untersuchen und unmittelbar erfasste Daten übertragen zu können. Alternativ zu den oben genannten Sensorarten können als Sensoren auch dotierte halbleitende Metalloxide, organische oder anorganische Beschichtungen auf Schwingquarzen oder Oberflächenwellenleitern, modifizierte leitfähige Polymere oder IR-Sensoren verwendet werden. Diese Aufzählung ist jedoch nicht abschließend.

Dabei ist erfindungsgemäß vorgesehen, dass die Sensoreinrichtung über einen Diffusionsabschnitt vom im Strömungskanal fließenden Medium getrennt ist, welcher Diffusionsabschnitt eine Gasdiffusion aus dem Medium zur Sensoreinrichtung hin zulässt.

Dies bietet den beachtlichen Vorteil, dass ein unmittelbarer Kontakt der Sensoreinrichtung mit dem Medium vermieden wird. Denn die Anordnung der Sensoreinrichtung bzw. der sensitiven Schicht direkt im Medium selbst, so dass diese unmittelbar mit diesem beaufschlagt wird, führt insbesondere im Langzeitbetrieb zu Problemen infolge der mitunter eintretenden Temperaturwechsel oder Druckwechsel in der Rohrleitung, und infolge von etwaigen Verunreinigungen oder Schwebstoffen, die die Sensoreinrichtung beschädigen oder zusetzen, so dass über längere Zeit die Sensoreinrichtung bzw. die sensitive Schicht zerstört wird. Wird jedoch, wie erfindungsgemäß vorgesehen, die Sensoreinrichtung über einen gaspermeablen Diffusionsabschnitt vom zu überwachenden Medium getrennt, ergibt sich kein direkter Kontakt. Die im Wasser befindlichen gelösten gasförmigen Stoffe oder Verbindungen, die es zu sensieren gilt, und bezüglich welcher die Sensoreinrichtung selektiv ist, diffundieren infolge ihres Dampfdrucks aus dem Medium, also aus dem Wasser, durch den Gasdiffusionsabschnitt hindurch zur Sensoreinrichtung hin, wo sie sensiert werden können. Gassensoreinrichtungen sind höchst sensitiv, sie sind in der Lage, bereits geringste Konzentrationen eines Stoffes oder einer Verbindung, bezüglich welcher die sensitive Schicht oder das sensitive Array ausgelegt ist, ermitteln zu können. Infolgedessen ist trotz Verwendung einer Gasdiffusionsschicht und damit einer echten Trennung der Sensoreinrichtung vom zu überwachenden Medium ein sicherer Nachweis auch geringster Konzentrationen sichergestellt. Dies gilt insbesondere, als üblicherweise in Rohrleitungen das Medium während weit über 90% der Zeit still steht, mithin also nicht strömt, so dass für etwaige Diffusionsprozesse hinreichend Zeit zur Verfügung steht.

Je nach Ausgestaltung der Sensoreinrichtung bzw. der sensitiven Schicht oder dergleichen der Sensoreinrichtung können unterschiedlichste Stoffe und Verbindungen ermittelt werden, beispielsweise Alkane, Alkohole, Aromate, Kohlenwasserstoffe, Stickstoffverbindungen, Schwefelverbindungen, Chlorverbindungen oder anorganische Stoffe. Bevorzugt kommt eine multifunktionale Sensoreinrichtung zum Einsatz, die auf eine Vielzahl unterschiedlicher Stoffe und Verbindungen sensitiv reagiert.

Die Einrichtung selbst kann ein separates, eigenständiges Bauteil sein, d.h., die Sensoreinrichtung ist in einem separaten Einrichtungsgehäuse, das zur Integration in einer Rohrleitung entsprechend ausgelegt ist, angeordnet. Eine besonders zweckmäßige Erfindungsalternative dazu sieht vor, die Sensoreinrichtung in einen in die Rohrleitung zu setzenden Durchfluss-, Wärme- oder Energiemengenzähler zu integrieren, wobei das Zählergehäuse dann das Einrichtungsgehäuse bildet, und wobei die Diffusionsschicht in einer den Strömungskanal des Mediums durch den Zähler begrenzenden Wand integriert ist. Die Sensoreinrichtung wird also zweckmäßigerweise in einen ohnehin der Erfassung von mediumbezogenen Parametern dienenden Zähler, der in einer Rohrleitung anzuordnen ist, integriert, d.h., man setzt auf bekannte Zählersysteme auf und erweitert diese um die integrierte Sensoreinrichtung. Erforderlich ist lediglich, einen entsprechenden Diffusionsabschnitt in einer den Strömungskanal durch den Zähler begrenzenden Wand vorzusehen und diesem Diffusionsabschnitt unmittelbar die Sensoreinrichtung nachzuschalten. Dies bietet ferner die Möglichkeit, die Übertragungseinrichtung, also das Kommunikationsmittel des Zählers zur Datenübertragung auch der Erfassungsdaten der Sensoreinrichtung zu nutzen. Üblicherweise sind moderne Zähler gleich welcher Art über entsprechende Kommunikationsmittel datentechnisch mit einer Datenempfangseinrichtung, sei diese stationär oder mobil, vernetzt, es existiert also eine netzwerkartige Datenkommunikationsstruktur. Entsprechende Kommunikationsstandards und Übertragungsprotokolle sind bereits hinreichend realisiert und im Einsatz. Die Funktionalität eines solchen Zählers kann nun erfindungsgemäß über die dort integrierte Sendeeinrichtung deutlich erweitert werden, darüber hinaus ist die Integration der Sensoreinrichtung einfach, nachdem lediglich auf bereits bekannte Kommunikationsstandards aufgesetzt wird. Mithin besteht also die Möglichkeit, unter Verwendung einer solchen multifunktionalen Einrichtung enthaltend einen Zähler nebst Sensoreinrichtung ein multifunktionales Netzwerk aufzubauen, das sowohl die Erfassung von mediumspezifischen Parametern über den Zähler als auch die gleichzeitige Überwachung des Mediums im Hinblick auf etwaige Gefahrenstoffe und eine sofortige Information hierüber ermöglicht.

Der Diffusionsabschnitt selbst kann in unterschiedlicher Weise ausgebildet sein. Zum einen kann der Diffusionsabschnitt Mikroöffnungen aufweisen, d.h., er ist mikroporös ausgeführt bzw. weist ein Mikrolochraster auf. Die Mikroöffnungen können dabei unmittelbar in einer den Strömungskanal begrenzenden Wand ausgebildet sein. Alternativ ist es auch denkbar, ein entsprechend mit Mikroöffnungen versehenes Diffusionselement einzusetzen. Diese Mikroöffnungen in der Wand oder im Diffusionselement können beispielsweise auf nasschemischen Weg über Ätzprozesse oder dergleichen erzeugt werden. Ein derartiges Diffusionselement kann in seiner Dicke beliebig ausgeführt werden, wobei natürlich die Dicke entscheidend die Diffusionsgeschwindigkeit beeinflusst, weshalb das Diffusionselement selbst möglichst dünn ausgeführt sein soll. Es ist selbstverständlich dicht in die Strömungskanalwand zu integrieren, um einen Wasserdurchtritt an dieser Stelle in den Bereich der nachgeschalteten Sensoreinrichtung bzw. Zählerelektronik zu vermeiden. In entsprechender Weise kann natürlich bei einer Ausbildung der Mikroporen direkt in der Strömungskanalwand der dortige Wandbereich zur Dicken-Verringerung entsprechend abgetragen sein, um auch hier einen relativ kurzen Diffusionsweg zu realisieren.

Alternativ zur Verwendung bzw. Ausbildung des Diffusionsabschnitts über Mikroöffnungen ist es auch denkbar, den Diffusionsabschnitt mittels einer organischen oder anorganischen Schicht, insbesondere in Form einer Keramik- oder Polymerschicht zu bilden, welche als Schicht in eine den Strömungskanal begrenzende Wand gesetzt ist. Als solche organischen oder anorganischen Diffusionsschichten können beliebige Schichten oder Materialien verwendet werden. Besonders bevorzugt sind Keramiken, insbesondere Aluminiumoxid-Keramik oder oxidkeramische Verbundstoffe mit hochporöser Matrix. Auch unterschiedlichste Polymere können zur Bildung der Polymerdiffusionsschicht verwendet werden. Lediglich exemplarisch seien EVA-Copolymere genannt, durch die beispielsweise aromatische Kohlenwasserstoffe oder Chlorkohlenwasserstoffe diffundieren. Selbstverständlich besteht die Möglichkeit, verschiedenartig ausgeführte Diffusionsabschnitte oder Diffusionsschichten zu verwenden, um lokal unterschiedliche Stoffdiffusionen zuzulassen, sofern der eine oder andere verwendete Diffusionswerkstoff eine nur selektive Diffusion zulässt. Diesen verschiedenen Diffusionsabschnitten kann eine gemeinsame Sensoreinrichtung zugeordnet sein, wie natürlich auch jedem Diffusionsabschnitt eine separate Sensoreinrichtung zugeordnet sein kann, denen dann bevorzugt eine gemeinsame Datenübertragungseinrichtung zugeordnet ist.

Die Sensoreinrichtung sollte zweckmäßigerweise möglichst einfach ausgeführt sein, da es primär lediglich darauf ankommt, überhaupt das Vorhandensein eines oder mehrerer Stoffe oder Verbindungen, die unerwünscht sind und bezüglich welcher die Sensoreinrichtung sensitiv ist, zu erfassen und eine zentrale Stelle darüber zu informieren. Denn bereits diese Information ist ausreichend, etwaige Maßnahmen zentral gesteuert einzuleiten. Eine tiefgehende Analyse, wie sie stationär in einem Labor erfolgt, ist mit der miniaturisierten Sensoreinrichtung nicht durchzuführen bzw. auch nicht erforderlich. Gleichwohl besteht selbstverständlich die Möglichkeit, den Komplexitätsgrad und damit die Analysetiefe der Sensoreinrichtung entsprechend zu erhöhen, um noch weitere Informationen bezüglich der sensierten Stoffe/Verbindungen über die Sensoreinrichtung zu ermitteln. Zweckmäßigerweise sind in der Sensoreinrichtung bzw. der ihr zugeordneten Steuerungseinrichtung entsprechende Grenzwerte abgelegt, wobei die Gabe der Erfassungsdaten an die Datenempfangseinrichtung in diesem Fall nur dann erfolgt, wenn der erfasste Ist-Wert einen zugeordneten Grenzwert übersteigt. Hierüber wird vermieden, dass eine Datenübertragung und damit die Einleitung etwaiger Schutzmaßnahmen bereits bei minimalsten Konzentrationen erfolgt. Selbstverständlich besteht im umgekehrten Fall die Möglichkeit, generell sämtliche Erfassungsdaten zu übertragen und die eigentliche Auswertung seitens der Datenempfangseinrichtung durchzuführen, mithin also dort erst den Ist-Wert-Grenzwert-Vergleich durchzuführen.

Die erfindungsgemäße Einrichtung ist insbesondere als eine Einrichtung bzw. in Verbindung mit einer Einrichtung zu verwenden, wie sie in der parallelen deutschen Patentanmeldung 10 2006 013 612.8-52 der Anmelderin, die am selben Tag wie die vorliegende Patentanmeldung eingereicht wurde, beschrieben ist. Die dortige Anmeldung betrifft ebenfalls eine Einrichtung zur Erfassung von Stoffen oder Verbindungen in einem strömenden Medium unter Verwendung einer Sensoreinrichtung und zeichnet sich insbesondere hinsichtlich der Datenkommunikation aus. Die Integration der Sensoreinrichtung in das jeweilige Gehäuse - sei es ein separates Gehäuse oder sei es das Zählergehäuse - kann in der Weise erfolgen, wie sie Gegenstand der vorliegenden Erfindung ist.

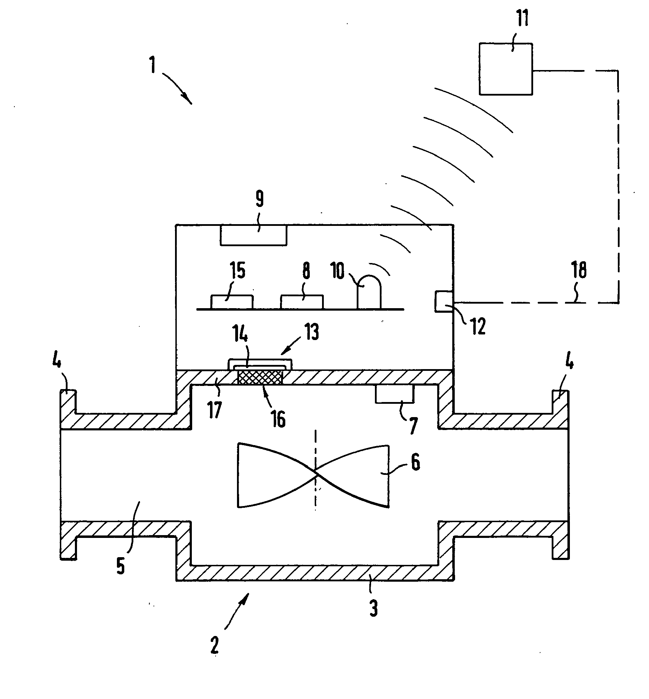

Weitere Vorteile, Merkmale und Einzelheiten der Erfindung ergeben sich aus dem im Folgenden beschriebenen Ausführungsbeispiel sowie anhand der Zeichnung.

In dieser ist eine erfindungsgemäße Einrichtung 1 gezeigt, bestehend aus einem Zähler 2, hier exemplarisch in Form eines Wasserzählers, der der Erfassung der Wasserdurchflussmenge dient. Der Zähler 2 weist ein Gehäuse 3 auf, das in bekannter Weise über seine Befestigungsflansche 4 in eine hier nicht näher gezeigte Rohrleitung gesetzt wird. Im Gehäuse 3 ist ein Strömungskanal 5 realisiert, in dem im gezeigten Beispiel ein Flügelrad 6 angeordnet ist, das über das strömende Wasser in Drehung versetzt wird. Diese Drehung wird über eine Erfassungseinrichtung 7 detektiert, die mit einer Steuerelektronik 8 in nicht näher gezeigter Weise kommuniziert. Seitens der Steuerelektronik 8 wird z.B. das Volumen ermittelt und entweder an einer Anzeigeeinrichtung 9 optisch angezeigt oder im gezeigten Beispiel über eine Übertragungseinrichtung 10, die hier als Funkeinrichtung ausgeführt ist, an eine externe Datenempfangseinrichtung 11 übertragen. Alternativ zur Funkübertragung oder zusätzlich hierzu kann auch ein Anschluss 12 für eine Datenleitung 18, beispielsweise eine ein- oder zweiadrige Leitung, vorgesehen sein, über die der Zähler 2 mit der Datenempfangseinrichtung 11 kabelgebunden gekoppelt ist.

Der grundsätzliche Aufbau und die Funktionsweise eines solchen Wasserzählers ist hinlänglich bekannt. Die Erfindung ist jedoch nicht auf den Einsatz eines Wasserzählers beschränkt. Vielmehr kann als Basisbauteil jedweder Zähler in Form eines Durchfluss-, Wärmemengen- oder Energiemengenzählers verwendet werden.

Erfindungsgemäß ist nun in diesem Zähler 2 eine Sensoreinrichtung 13 integriert. Diese Sensoreinrichtung 13 weist einen sensorisch aktiven Abschnitt 14 auf, beispielsweise in Form eines Sensorarrays, das verschiedene aktive Zonen, die bezüglich unterschiedlicher Stoffe oder Verbindungen sensitiv reagieren, ausgeführt ist. Ferner ist ihr eine Steuerelektronik 15 zugeordnet, die jedoch auch unmittelbarer Teil der Sensoreinrichtung 13 sein kann. Bevorzugt ist die Sensoreinrichtung 13 lediglich als kleindimensionierter Sensorchip ausgeführt, der alle erforderlichen Komponenten, nämlich Sensorabschnitt 14 und Steuerelektronik 15 und ggf. eine separate Leistungsversorgung beinhaltet.

Die Sensoreinrichtung 13 kommuniziert mit dem im Strömungskanal 5 befindlichen Medium über einen gaspermeablen Diffusionsabschnitt 16, der in der Wand 17, die den Strömungskanal 5 begrenzt, integriert ist. Dieser Diffusionsabschnitt kann in unterschiedlichster Weise ausgeführt sein, in jedem Fall lässt er die Diffusion gasförmig gelöster Stoffe oder Verbindungen aus dem Medium, hier dem Wasser, zur Sensoreinrichtung 13 zu. Der Diffusionsabschnitt 16 kann beispielsweise in Form einer Keramikschicht, die nach Art eines Inlays mit möglichst geringer Wandstärke in die Strömungskanalwand 17 eingesetzt ist oder als Polymerschicht, die ebenfalls hinreichend dünn, jedoch noch ausreichend stabil ist, ausgeführt sein kann. Denkbar ist auch der Einsatz eines Mikroöffnungen oder einen Microlochraster aufweisenden Diffusionselements oder die Ausbildung des Diffusionsabschnitts unmittelbar in der Gehäusewand selbst. Die Dicke des Diffusionsabschnitts sollte möglichst gering sein, um den Diffusionsweg der aufgrund ihres Dampfdrucks aus dem Wasser abdampfenden Stoffe/Verbindungen zum gassensitiven Abschnitt 14 der Sensoreinrichtung 13 möglichst kurz zu halten. Denn je dicker der Diffusionsabschnitt ist, umso größer ist die Diffusionszeit. Aus Übersichtlichkeitsgründen ist der Diffusionsabschnitt 16 in der Figur relativ stark dargestellt, tatsächlich sollte er bevorzugt ≤ 5 mm, insbesondere ≤ 3 mm in seiner Dicke sein.

Der Diffusionsabschnitt selbst, sofern es sich bei ihm um ein separates Bauteil handelt, kann beispielsweise in eine entsprechende Öffnung der Strömungskanalwand eingeklebt werden, um eine sichere, dichte Integration zu realisieren. Der Diffusionsabschnitt selbst besteht beispielsweise aus einer Halterung, in der das die Diffusion erlaubende gaspermeable Material aufgenommen ist, über welche Halterung der Diffusionsabschnitt beispielsweise eingeklebt werden kann, alternativ wäre es auch denkbar, die Halterung derart auszugestalten, dass der Diffusionsabschnitt verschraubt oder verlötet oder dergleichen werden kann.

Diffundieren nun Stoffe oder Verbindungen aus dem Wasser durch den Diffusionsabschnitt 16 zur Sensoreinrichtung 13 und ist der Sensorabschnitt 14 bezüglich eines oder mehrerer dieser Stoffe oder Verbindungen sensitiv, so wird seitens der Steuerelektronik 15 ein entsprechendes Erfassungssignal oder ein entsprechendes Erfassungsdatum erzeugt, das unter Verwendung der Übertragungseinrichtung 10 oder über den Übertragungsanschluss 12 an die Datenempfangseinrichtung 11 übertragen wird. Die Übertragung kann dabei abhängig davon sein, ob der ermittelte Ist-Konzentrationswert des erfassten Stoffes oder der erfassten Verbindung oberhalb eines in der Steuerelektronik 15 abgelegten Grenzwerts ist, da nur dann von einer hinreichenden, eine Reaktion erfordernden Stoff- oder Verbindungskonzentration im Wasser auszugehen ist. Bei niedrigeren Konzentrationen unterbleibt die Erzeugung eines Erfassungssignals, es erfolgt keine Datenübertragung. Bei dieser Ausgestaltung ist also die Sensoreinrichtung 13 bzw. die ihr zugeordnete Steuerelektronik auch zur Erfassung der Stoff- oder Verbindungskonzentration ausgebildet.

Die Leistungsversorgung der Sensoreinrichtung 13 nebst zugeordneter Steuerelektronik 15 erfolgt zweckmäßigerweise über die ohnehin zählerseitig integrierte Leistungsversorgung, so dass eine separate Leistungsversorgung nicht vorzusehen ist.

Abschließend ist festzuhalten, dass die Erfindung gleichermaßen eine Einrichtung betrifft, die lediglich aus einem spezifischen Einrichtungsgehäuse mit integrierter Sensoreinrichtung besteht, bei der also die Sensoreinrichtung nicht in einen Zähler integriert ist. In diesem Fall weist das spezifische Einrichtungsgehäuse ebenfalls entsprechende Anschlussmittel zum Integrieren in eine Rohrleitung aus, im Gehäuse ist ebenfalls ein Strömungskanal ausgebildet, in den ein Diffusionsabschnitt integriert oder in dem ein solcher vorgesehen ist. Ferner ist eine Sensoreinrichtung nebst Steuerelektronik in der beschriebenen Ausgestaltung integriert, wie auch eine entsprechende Übertragungseinrichtung zum Übertragen der Erfassungsdaten. In diesem Fall ist zweckmäßigerweise auch eine integrierte Leistungsversorgung vorzusehen. Diese Einrichtung stellt also ein separates Bauteil zur in der Figur gezeigten integrierten Lösung dar.

## Patentansprüche

1. Einrichtung zur Erfassung des Vorhandenseins eines oder mehrerer Stoffe oder Verbindungen in einem in einer Rohrleitung strömenden Medium, insbesondere Wasser, **gekennzeichnet durch** eine der Erfassung der Stoffe oder Verbindungen dienende Sensoreinrichtung (13), die in einem in die Rohrleitung zu setzenden Gehäuse (3) angeordnet ist, und von einem das Medium führenden Strömungskanal (5) über einen eine Gasdiffusion aus dem Medium erlaubenden Diffusionsabschnitt (16) getrennt ist.

2. Einrichtung nach Anspruch 1, **dadurch gekennzeichnet, dass** die Sensoreinrichtung (13) in einen in die Rohrleitung zu setzenden Durchfluss-, Wärme- oder Energiemengenzähler (2) integriert ist, wobei der Diffusionsabschnitt (16) in einer den Strömungskanal (5) des Mediums durch den Zähler begrenzenden Wand (17) integriert ist.

3. Einrichtung nach Anspruch 1 oder 2, **dadurch gekennzeichnet, dass** der Diffusionsabschnitt (16) die Diffusion ermöglichende Mikroöffnungen aufweist.

4. Einrichtung nach Anspruch 3, **dadurch gekennzeichnet, dass** der Mikroöffnungen unmittelbar in einer den Strömungskanal (5) begrenzenden Wand (17) ausgebildet ist, oder dass in die Wand (17) ein die Mikroöffnungen aufweisendes Diffusionselement eingesetzt ist.

5. Einrichtung nach Anspruch 1 oder 2, **dadurch gekennzeichnet, dass** der Diffusionsabschnitt (16) mittels einer organischen oder anorganischen Schicht, insbesondere in Form einer Keramik- oder Polymerschicht gebildet ist, die in eine den Strömungskanal (5) begrenzenden Wand (17) eingesetzt ist.

6. Einrichtung nach einem der vorangehenden Ansprüche, **dadurch gekennzeichnet, dass** der Sensoreinrichtung (13) eine Datenübertragungseinrichtung (10, 12) zugeordnet ist, über die die Erfassungsdaten an eine externe Datenempfangseinrichtung (11) übertragbar sind.

7. Einrichtung nach Anspruch 6, wobei die Sensoreinrichtung in einem Zähler (2) integriert ist, **dadurch gekennzeichnet, dass** die Datenübertragungseinrichtung (10, 12) die des Zählers (2) ist.
